# EUROPÄISCHE PATENTSCHRIFT

(11) **EP 3 899 520 B1**
(45) Veröffentlichungstag und Bekanntmachung des Hinweises auf die Patenterteilung: **14.06.2023**
(21) Anmeldenummer: 19806125.1
(22) Anmeldetag: 12.11.2019
(51) Int. Cl.: G01N 27/417, G01N 27/419, G01N 33/00

(54) **VERFAHREN ZUR VERRINGERUNG VON MESSFEHLERN BEI DER ERFASSUNG VON AMMONIAK BEIM BETREIBEN EINES SENSORSYSTEMS**
METHOD FOR REDUCING MEASUREMENT ERRORS WHEN DETECTING AMMONIA WHEN OPERATING A SENSOR SYSTEM
PROCÉDÉ DE RÉDUCTION D'ERREURS DE MESURE LORS DE LA DÉTECTION D'AMMONIAC LORS DU FONCTIONNEMENT D'UN SYSTÈME DE CAPTEUR

(30) Priorität: 20.12.2018 DE 102018222624
(43) Veröffentlichungstag der Anmeldung: 27.10.2021
(73) Patentinhaber: Robert Bosch GmbH, 70442 Stuttgart (DE)
(72) Erfinder: WAHL, Thomas, 75181 Pforzheim (DE)
(86) Internationale Anmeldenummer: PCT/EP2019/081040
(87) Internationale Veröffentlichungsnummer: WO 2020/126231

(56) Entgegenhaltungen:
- DE-A1-102007 052 754
- DE-A1-102016 202 218
- US-A1- 2009 139 210

## Beschreibung

### Stand der Technik

Aus dem Stand der Technik ist eine Vielzahl von Verfahren und Sensoren zum Nachweis mindestens eines Anteils der Messgaskomponente mit gebundenem Sauerstoff in einem Gasgemisch, insbesondere in einem Abgas einer Verbrennungskraftmaschine, durch Erfassen eines Anteils an Sauerstoff, der durch eine Reduktion der Messgaskomponente mit dem gebundenem Sauerstoff erzeugt wird, bei Anwesenheit von molekularem Sauerstoff bekannt.

Sensoren zum Nachweis mindestens eines Anteils der Messgaskomponente mit gebundenem Sauerstoff in einem Gasgemisch, die auch verkürzt oder vereinfacht NOₓ-Sensoren oder Stickoxid-Sensoren bezeichnet werden, sind beispielsweise in Reif, K., Deitsche, K-H. et al., Kraftfahrtechnisches Taschenbuch, Springer Vieweg, Wiesbaden, 2014, Seite 1338-1347 beschrieben.

Stickoxid-Sensoren (= NOₓ-Sensoren), die heutzutage in der Automobiltechnik eingesetzt werden, funktionieren nach dem Grenzstromprinzip, analog zu Sauerstoff-Sensoren, wie beispielsweise Lambda Sensoren. Ein solcher Stickoxid-Sensor umfasst eine Nernst-Konzentrationszelle, die auch Referenzzelle genannt wird, eine modifizierte Sauerstoffpumpzelle und eine weitere modifizierte Sauerstoffpumpzelle, die die sogenannte NOₓ-Zelle. Eine dem Abgas ausgesetzte äußere Pumpelektrode und eine innere Pumpelektrode in einem ersten Hohlraum, der vom Abgas durch eine Diffusionsbarriere getrennt ist, bilden die Sauerstoffpumpzelle. Im ersten Hohlraum befindet sich auch die Nernstelektrode und in einem Referenzgasraum die Referenzelektrode, die zusammen die Nernstzelle bilden. Die NOₓ-Zelle umfasst eine NOₓ-Pumpelektrode und eine Gegenelektrode. Die NOₓ-Pumpelektrode befindet sich einem zweiten Hohlraum, der mit dem ersten inneren Hohlraum verbunden und von diesem durch eine Diffusionsbarriere getrennt ist. Die Gegenelektrode befindet sich in dem Referenzgasraum. Alle Elektroden in dem ersten und zweiten Hohlraum haben einen gemeinsamen Rückleiter.

Bei Betrieb des Stickoxid-Sensor wird der sogenannten O2-Zelle der Sauerstoff aus dem ersten Hohlraum, der über eine Diffusionsbarriere mit dem Abgas verbunden ist, entfernt. Der dadurch resultierende Pumpstrom ist dann proportional zum Sauerstoffgehalt der Umgebungsluft im Messgas- bzw. Abgasstrom. In der NOₓ-Zelle werden die Stickoxide abgepumpt. Das Stickoxid NOₓ in der in dem zweiten Hohlraum befindlichen Atmosphäre, wird durch Anlegen einer konstanten Pumpspannung reduziert bzw. abgebaut. Der durch Reduktion oder Abbau der Messgaskomponente in dem zweiten Hohlraum erzeugte Sauerstoff, der vorzugsweise aus der Reduktion des Stickoxids NOₓ stammt, wird in einen Referenzgasraum abgepumpt. So hat die angelegte Pumpspannung gegen den Widerstand der NOₓ-Zelle und durch die Konzentration des Stickoxids NOₓ bzw. Sauerstoffs einen Pumpstrom zur Folge, der proportional zum Gehalt an Stickoxid NOₓ bzw. Sauerstoff ist und das NOₓ-Messsignal darstellt.

Um in modernen Automobilen mit Dieselmotorantrieb hohe Reinigungsgrade von NO und NO₂ zu erreichen, wird die sogenannte SCR-Technik (SCR = selektive catalytic reduction) eingesetzt. Dabei wird eine wässrige Harnstofflösung vor dem SCR-Katalysator in das Abgasrohr eingespritzt. Dieser Harnstoff zersetzt sich bei Abgastemperaturen auf dem Katalysator zu Ammoniak (NH₃) und Kohlenstoffdioxid (CO₂). Der Ammoniak reagiert am Katalysator mit NO und NO₂ zu molekularem Stickstoff N₂. Für die Effizienz der Abgasreinigung ist es dabei von Vorteil, die NH₃-Konzentration selektiv neben der NO- und NO₂-Konzentration an einer Position in einem Katalysator bzw. zwischen zwei Katalysatoren oder hinter einem SCR-Katalysator zu messen. Bekannte Stickoxid-Sensoren, wie sie beispielsweise in Reif, K., Deitsche, K-H. et al., Kraftfahrtechnisches Taschenbuch, Springer Vieweg, Wiesbaden, 2014, Seite 1338-1347 beschrieben sind, sind in Zirkondioxid-Technologie aufgebaut sind. Diese enthalten als zentrales Messmittel ein keramisches Sensorelement, das durch einen integrierten Heizer auf eine feste Betriebstemperatur im Bereich von 650 bis 850 °C beheizt wird. Dadurch wird das dotierte Zirkondioxid zu einem elektrischen 02-lonenleiter. Der Aufbau der Messzellen folgt der bekannten Aufbaustruktur nach dem Doppelkammer-Prinzip. Diese Sensoren messen NO, NO₂ und NH₃ als Summensignal.

Nun lässt sich ein NO/NO₂-messender Sensor mit weiteren Messzellen ausrüsten, um weitere Gaskomponenten zu messen. Besonders vorteilhaft ist eine Ausrüstung mit einer oder mehrerer Ammoniak-messenden Messzellen. Der Sensor kann für eine verbesserte Regelung in einem SCR-System eingesetzt werden. Durch die Bereitstellung einer weiteren Messgröße wird die Regelung für eine hocheffiziente Abgasreinigung erleichtert.

Trotz der Vorteile der aus dem Stand der Technik bekannten Sensorsysteme mit einer ersten Messeinheit oder Messzelle zum Erfassen von Stickoxid und einer zweiten Messeinheit oder Messzelle zum Erfassen von Ammoniak sowie Verfahren zum Betreiben desselben, beinhalten diese noch Verbesserungspotenzial. Messzellen, die NH₃ in Abgasen nachweisen können, sind beispielsweise mit Mischpotenzialelektroden aufgebaut, die gegen eine Gleichgewichtselektrode, z.B. aus Platin hergestellt, ein elektrochemisches Potenzial erzeugen. Für Mischpotenzialelektroden ist bekannt, dass sie über die Betriebszeit und im Abgas einer Alterung unterliegen. Das führt dazu, dass das Signal sich über die Betriebsdauer verändert, in der Regel sich verringert. Dadurch können Messfehler nicht selten bis 50% entstehen.

Ein derartiger Sensor ist auch aus der DE 10 2016 202 218 A1 bekannt.

### Offenbarung der Erfindung

Es wird daher ein Verfahren zum Betreiben eines Sensorsystems zum Nachweis mindestens eines Anteils einer Messgaskomponente mit gebundenem Sauerstoff in einem Messgas vorgeschlagen, welches die Nachteile bekannter Verfahren zum Betreiben dieser Sensorsysteme zumindest weitgehend vermeidet und bei dem die Messfehler bei der Erfassung von Ammoniak durch Elektrodenalterung deutlich verringert bzw. vermieden werden können.

Ein erfindungsgemäßes Verfahren ist in Anspruch 1 beschrieben.

Ein Grundgedanke ist, die bekannte Empfindlichkeit des NOx-Sensors auf NH₃ für Ermittlung eines Korrekturwerts zu nutzen. So kann für vorab bestimmte Betriebszustände des Motors und damit des Abgassystems bzw. aus Plausibilisierungen verschiedener Informationen im Motorsteuergerät abgeleitet werden, ob im Abgasstrang NO und NO₂ vorliegen. Kann dies ausgeschlossen werden bzw. liegen diese in nur vernachlässigbaren Mengen vor und wird gleichzeitig ein Signal am NOx (NOx = NO +NO₂) messenden Sensorteil sowie gleichzeitig am NH₃-messenden Sensorteil detektiert, ist davon auszugehen, dass es sich um NH₃ handelt. Nun kann das Signal aus dem NH₃-messenden Sensorteil mit jenem des NOx-messenden Sensorteils verglichen werden. Weicht das NH₃-Kennlinien-Äquivalent des NH₃-Sensor-Signals vom NH₃-Kennlinien-Äquivalent des NOx-Sensors ab, wird ein Abweichungsfaktor ermittelt, ein Korrekturwert berechnet und ab sofort als Korrekturfaktor auf das NH₃-Signal angewendet. Damit lassen sich in einfacher Weise Messfehler der NH₃-Messeinheit bzw. Messzelle verringern bzw. vermeiden.

Bei einer Weiterbildung ist die vorbestimmte Bedingung ein Unterschreiten eines Schwellwerts eines Anteils an Stickoxid in dem Messgas. Entsprechend wird die Korrekturfunktion dann durchgeführt, beispielsweise in einem Motorsteuergerät oder in einem Sensorsteuergerät, wenn ein Betriebszustand identifiziert wird, beispielsweise durch das Motorsteuergerät, bei dem kein NOx oder nur vernachlässigbare Mengen an NOx im Abgasstrang vorliegen.

Erfindungsgemäß ist der Schwellwert für Stickoxid 7% eines Anteils an Ammoniak in dem Messgas. Dies stellt den Schwellwert dar, bis zu dem das Verfahren sicher durchführbar ist.

Bei einer Weiterbildung wird der Korrekturwert durch Division des zweiten Messsignalwerts mit dem ersten Messsignalwert und anschließendem Bilden des Kehrwerts gebildet. Damit kann der Korrekturwert ohne großen Aufwand gebildet werden.

Bei einer Weiterbildung wird das zweite korrigierte Messsignal durch Multiplizieren des zweiten Messsignals mit dem Korrekturwert gebildet. Damit kann das zweite Messsignal in einfacher Weise korrigiert werden.

Bei einer Weiterbildung wird der Korrekturwert nur gebildet, falls der Abweichungswert des zweiten Messsignalwerts von dem ersten Messsignalwert einen vorbestimmten Schwellwert für den Abweichungswert, insbesondere einen Anteil zwischen 10% und 50% des Signals und bevorzugt zwischen 10% und 25%, überschreitet. Damit wird sichergestellt, dass eine Korrektur nur aufgrund erheblicher Alterungsfehler der Elektroden erfolgt.

Bei einer Weiterbildung sind die erste Messeinheit und die zweite Messeinheit in einem einzigen Sensorelement angeordnet. Beispielsweise wird ein Stickoxid-Sensor, wie er aus dem oben genannten Stand der Technik bekannt ist, um eine weitere Messeinheit bzw. Messzelle erweitert.

Alternativ ist die erste Messeinheit in einem ersten Sensorelement angeordnet und die zweite Messeinheit in einem zweiten Sensorelement angeordnet ist, wobei sich das erste Sensorelement von dem zweiten Sensorelement unterscheidet, wobei das erste Sensorelement und das zweite Sensorelement in einem einzigen Sensor angeordnet sind. Mit anderen Worten werden zwei getrennte Sensorelemente in einem einzigen Sensor verbaut.

Alternativ ist die erste Messeinheit in einem ersten Sensorelement angeordnet und ist die zweite Messeinheit in einem zweiten Sensorelement angeordnet, wobei sich das erste Sensorelement von dem zweiten Sensorelement unterscheidet, wobei das erste Sensorelement in einem ersten Sensor angeordnet ist und das zweite Sensorelement in einem zweiten Sensor angeordnet ist, wobei sich der erste Sensor von dem zweiten Sensor unterscheidet, wobei der erste Sensor und der zweite Sensor benachbart zueinander in dem Messgas angeordnet werden. Mit anderen Worten sind der erste Sensor und zweite Sensor physisch getrennt aufgebaute Sensoren, die benachbart zueinander dem zu messenden Gasgemisch ausgesetzt werden.

Bei einer Weiterbildung ist das Messgas ein Abgas einer Verbrennungskraftmaschine, wobei das Sensorsystem mit einem Motorsteuergerät der Verbrennungskraftmaschine verbunden wird, wobei das Verfahren durchgeführt wird, wenn das Motorsteuergerät ein Triggersignal an das Sensorsystem sendet. Damit erfolgt die Durchführung des Verfahrens nur auf Anforderung des Motorsteuergeräts, so dass sichergestellt ist, dass die vorbestimmte Bedingung vorliegt.

Bei einer Weiterbildung ist das Messgas ein Abgas einer Verbrennungskraftmaschine, wobei das korrigierte zweite Messsignal zur Steuerung oder Regelung einer in das Abgas eingespritzten Menge an Harnstofflösung verwendet wird. Beispielsweise wird die Korrekturfunktion in einem SCR-Dosiersteuergerät zur Steuerung oder Regelung der Harnstofflösungseinspritzmenge durchgeführt.

Schließlich betrifft die Erfindung auch ein Sensorsystem nach Anspruch 10.

Unter einem Festelektrolyten ist im Rahmen der vorliegenden Erfindung ein Körper oder Gegenstand mit elektrolytischen Eigenschaften, also mit Ionen leitenden Eigenschaften, zu verstehen. Insbesondere kann es sich um einen keramischen Festelektrolyten handeln. Dies umfasst auch das Rohmaterial eines Festelektrolyten und daher die Ausbildung als so genannter Grünling oder Braunling, der erst nach einem Sintern zu einem Festelektrolyten wird. Insbesondere kann der Festelektrolyt als Festelektrolytschicht oder aus mehreren Festelektrolytschichten ausgebildet sei. Unter einer Schicht ist im Rahmen der vorliegenden Erfindung eine einheitliche Masse in flächenhafter Ausdehnung einer gewissen Höhe zu verstehen, die über, unter oder zwischen anderen Elementen liegt.

Unter einer Elektrode ist im Rahmen der vorliegenden Erfindung allgemein ein Element zu verstehen, welches in der Lage ist, den Festelektrolyten derart zu kontaktieren, dass durch den Festelektrolyten und die Elektrode ein Strom aufrechterhalten werden kann. Dementsprechend kann die Elektrode ein Element umfassen, an welchem die Ionen in den Festelektrolyten eingebaut und/oder aus dem Festelektrolyten ausgebaut werden können. Typischerweise umfassen die Elektroden eine Edelmetallelektrode, welche beispielsweise als Metall-Keramik-Elektrode auf dem Festelektrolyten aufgebracht sein kann oder auf andere Weise mit dem Festelektrolyten in Verbindung stehen kann. Typische Elektrodenmaterialien sind Platin-Cermet-Elektroden. Auch andere Edelmetalle, wie beispielsweise Gold oder Palladium, sind jedoch grundsätzlich einsetzbar.

Unter einem Heizelement ist im Rahmen der vorliegenden Erfindung ein Element zu verstehen, das zum Erwärmen des Festelektrolyten und der Elektroden auf mindestens ihre Funktionstemperatur und vorzugsweise auf ihre Betriebstemperatur dient. Die Funktionstemperatur ist diejenige Temperatur, ab der der Festelektrolyt für Ionen leitend wird und die ungefähr 350 °C beträgt. Davon ist die Betriebstemperatur zu unterscheiden, die diejenige Temperatur ist, bei der das Sensorelement üblicherweise betrieben wird und die höher ist als die Funktionstemperatur. Die Betriebstemperatur kann beispielsweise von 500 °C bis 950 °C sein. Das Heizelement kann einen Heizbereich und mindestens eine Zuleitungsbahn umfassen. Unter einem Heizbereich ist im Rahmen der vorliegenden Erfindung der Bereich des Heizelements zu verstehen, der in dem Schichtaufbau entlang einer zu der Oberfläche des Sensorelements senkrechten Richtung mit einer Elektrode überlappt. Üblicherweise erwärmt sich der Heizbereich während des Betriebs stärker als die Zuleitungsbahn, so dass diese unterscheidbar sind. Die unterschiedliche Erwärmung kann beispielsweise dadurch realisiert werden, dass der Heizbereich einen höheren elektrischen Widerstand aufweist als die Zuleitungsbahn. Der Heizbereich und/oder die Zuleitung sind beispielsweise als elektrische Widerstandsbahn ausgebildet und erwärmen sich durch Anlegen einer elektrischen Spannung. Das Heizelement kann beispielsweise aus einem Platin-Cermet hergestellt sein.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
- Figur 1: einen prinzipiellen Aufbau eines erfindungsgemäßen Sensorsystems,
- Figur 2: ein Flussdiagramm eines erfindungsgemäßen Verfahrens

### Ausführungsformen der Erfindung

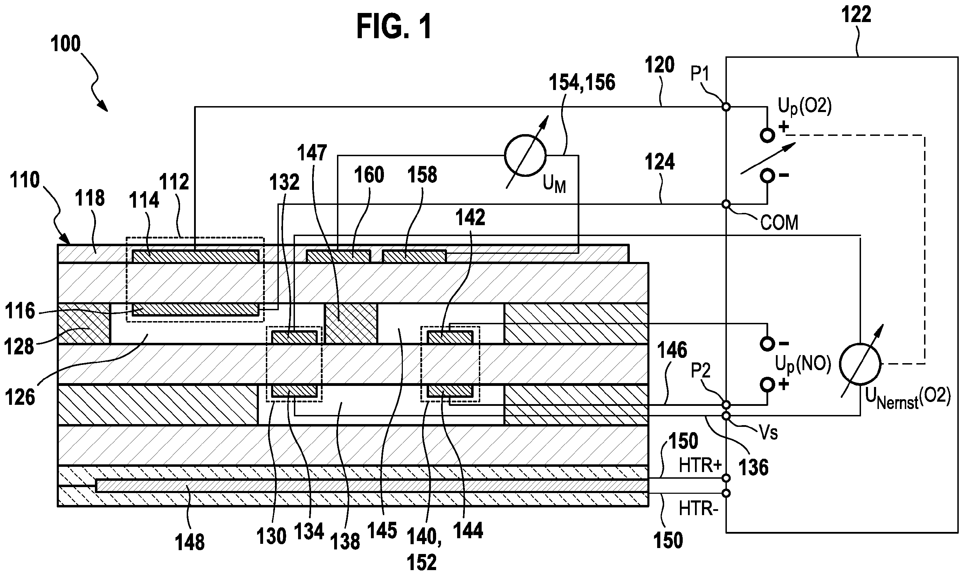

Figur 1 zeigt einen prinzipiellen Aufbau eines erfindungsgemäßen Sensorsystems 100, welches zur Durchführung des erfindungsgemäßen Verfahrens besonders geeignet ist. Das Sensorsystem 100 ist zum Nachweis mindestens eines Anteils einer Messgaskomponente mit gebundenem Sauerstoff, im Folgenden beispielhaft als Stickoxid NOx bezeichnet, in einem Gasgemisch, beispielhaft einem Abgas einer Verbrennungskraftmaschine, eingerichtet.

Das Sensorsystem 100 umfasst hierzu ein erstes Sensorelement 110. Das erste Sensorelement 110 umfasst eine erste Pumpzelle 112, welche zwischen einer äußeren Pumpelektrode 114 und einer inneren Pumpelektrode 116 ausgebildet ist. Die äußere Pumpelektrode 114, welche mittels einer porösen Aluminiumoxidschicht 118 von der Umgebung des ersten Sensorelements 110 getrennt ist, verfügt hierbei über eine erste elektrisch leitende Verbindung 120, über welche sich ein erster Pumpstrom I_{P1} in der ersten Pumpzelle 112 erzeugen lässt. Die erste elektrisch leitende Verbindung 120 ist hierzu mit einem Anschluss P1 eines externen elektronischen Steuergeräts 122 verbunden. Um einen vollständigen Stromkreis zu erhalten, verfügt die innere Pumpelektrode 116 ebenfalls über eine zweite elektrisch leitende Verbindung 124, welche zu einem gemeinsamen Anschluss COM des externen elektronischen Steuergeräts 122 führt. Die erste Pumpzelle 112 liegt an einem ersten Hohlraum 126 an, der sich im Inneren des ersten Sensorelements 110 befindet und mit dem Messgas in Verbindung steht. Durch Erzeugen des ersten Pumpstroms I_{P1} in der ersten Pumpzelle 112 lässt sich ein erster Anteil von Sauerstoffionen, welche aus molekularem Sauerstoff aus dem Gasgemisch gebildet werden, zwischen dem ersten Hohlraum 126 und der Umgebung des Sensors 100 transportieren. In dem Eintrittsweg aus der Umgebung zu dem ersten Hohlraum 126 ist eine Diffusionsbarriere 128 vorhanden.

Das erste Sensorelement 110 weist weiterhin eine elektrische Nernstzelle 130 auf, welche eine Nernst-Elektrode 132 und eine Referenzelektrode 134 aufweist. Während die Nernst-Elektrode 132 über die zweite elektrisch leitende Verbindung 124 zusammen mit der inneren Pumpelektrode 116 zu dem gemeinsamen Anschluss COM verfügt, weist die Referenzelektrode 134 eine gesonderte elektrisch leitende Verbindung 136 zu einem Anschluss Vs des externen elektronischen Steuergeräts 122 für die Nernstspannung Vs auf. Die Nernstzelle 130 liegt an einem Referenzgasraum 138 an. Ein zweiter Anteil der Sauerstoffionen aus dem Messgasraum 126 und/oder aus der Umgebung des Sensors 100 wird in den Referenzgasraum 138 durch Anlegen eines Referenz-Pumpstroms zwischen dem Anschluss Vs und dem gemeinsamen Anschluss COM transportiert. Hierbei wird der Wert für den Referenz-Pumpstrom derart eingestellt, dass sich ein festgelegter Anteil der Sauerstoffionen in dem Referenzgasraum 138 ausbildet. Vorzugsweise wird in diesem Zusammenhang auch der Wert für den ersten Pumpstrom I_{P1} derart eingestellt, dass sich ein festgelegtes Verhältnis zwischen dem ersten Anteil der Sauerstoffionen in dem Messgasraum 126 und dem zweiten Anteil der Sauerstoffionen in dem Referenzgasraum 138 ergibt.

Die in dem Gasgemisch weiterhin enthaltene Messgaskomponente Stickoxid NOₓ mit dem gebundenen Sauerstoff gelangt, insbesondere durch Diffusion, weitgehend unbeeinflusst in eine zweite Pumpzelle 140 des ersten Sensorelements 110, welche auch als "NOₓ-Pumpzelle" bezeichnet werden kann. Die zweite Pumpzelle 140 weist eine NOₓ-Pumpelektrode 142 und eine NOₓ-Gegenelektrode 144 auf und liegt an einem zweiten Hohlraum 145 im inneren des ersten Sensorelements 110 an. Wenigstens eine der beiden Elektroden NOₓ-Pumpelektrode 142 und/oder NOₓ-Gegenelektrode 144 sind derart ausgestaltet, dass bei Anlegen einer Spannung mittels Katalyse aus der Messgaskomponente NOₓ weiterer molekularer Sauerstoff erzeugt werden kann, welcher in der zweiten Pumpzelle 140 gebildet wird.

Während die NOₓ-Pumpelektrode 142 eine elektrisch leitende Verbindung 146 aufweist, welche zu dem gemeinsamen Anschluss COM führt, weist die NOₓ-Gegenelektrode 144 eine elektrisch leitende Verbindung 146 auf, über welche ein zweiter Pumpstrom I_{P2} an die zweite Pumpzelle 140 angelegt werden kann. Die elektrisch leitende Verbindung 146 ist hierzu mit einem Anschluss P2 des externen elektronischen Steuergeräts 122 verbunden. Bei Anlegen eines zweiten Pumpstroms I_{P2} an die zweite Pumpzelle 140 wird ein Anteil von weiteren Sauerstoffionen, welche aus dem weiteren molekularen Sauerstoff gebildet wurden, in den Referenzgasraum 138 transportiert. Der zweite Hohlraum 145 ist von dem ersten Hohlraum 126 durch eine Diffusionsbarriere 147 getrennt. Das erste Sensorelement 110 verfügt weiterhin über ein Heizelement 148, welches mittels zweier Zuleitungen 150 mit Anschlüssen HTR+ und HTR- des Steuergeräts 122 verbunden ist, über welche ein Heizstrom in das Heizelement 148 eingebracht werden kann, welches mittels Erzeugen einer Heizleistung das erste Sensorelement 110 auf die gewünschte Temperatur bringen kann. Die zweite Pumpzelle 140 ist als Messzelle oder Messeinheit 152 zur Erfassung von Stickoxid in dem Messgas ausgebildet.

Das Sensorsystem 100 umfasst weiterhin ein zweites Sensorelement 154. Das zweite Sensorelement 154 umfasst eine zweite Messzelle oder Messeinheit 156 zum Erfassen von Ammoniak in dem Messgas. Die zweite Messeinheit 156 weist zu diesem Zweck mindestens eine Mischpotenzialelektrode 158 und eine Gleichgewichtselektrode 160 auf. Die Gleichgewichtselektrode 160 ist beispielsweise aus Platin hergestellt. Die Mischpotenzialelektrode 158 erzeugt gegen die Gleichgewichtselektrode 160 ein elektrochemisches Potenzial. Das zweite Sensorelement 154 kann mit dem elektronischen Steuergerät verbunden sein. Alternativ ist das zweite Sensorelement 154 mit einem eigenen Steuergerät verbunden.

Wie in Figur 1 zu erkennen, ist das zweite Sensorelement 154 in dem ersten Sensorelement 110 angeordnet, so dass die erste Messeinheit 152 und die zweite Messeinheit 156 integriert sind. Es wird explizit betont, dass das erste Sensorelement 110 und das zweite Sensorelement 154 getrennt voneinander vorliegen können. Es wird weiterhin explizit betont, dass das zweite Sensorelement 154 und das erste Sensorelement 110 in einem Sensor angeordnet sein können und beispielsweise von einem gemeinsamen Sensorgehäuse umgeben sind. Es wird weiterhin explizit betont, dass die erste Messeinheit 152 und die zweite Messeinheit 156 in voneinander getrennten Sensoren angeordnet sein können, die benachbart zueinander dem Messgas ausgesetzt werden.

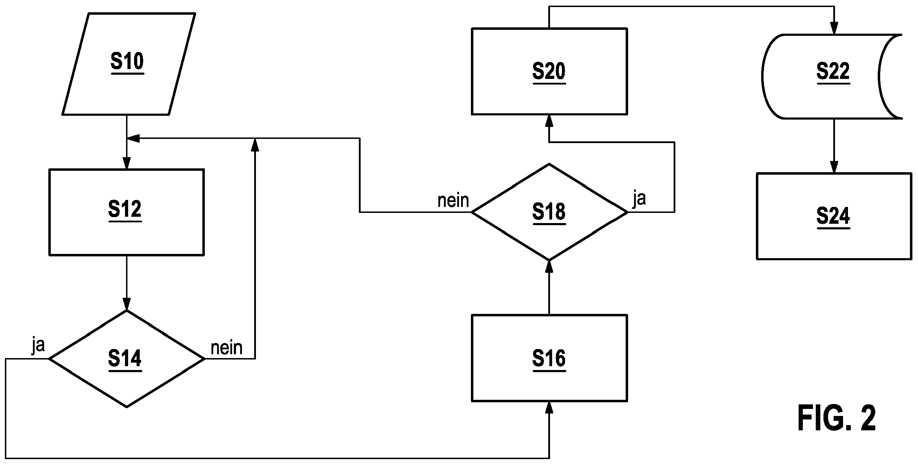

Figur 2 zeigt Flussdiagramm eines erfindungsgemäßen Verfahrens zum Betreiben des Sensors 100. Zunächst werden in Schritt S10 Daten gesammelt, die Informationen über das Messgas enthalten oder basierend auf denen Informationen über das Messgas gewonnen werden können. Bei dem gezeigten Ausführungsbeispiel werden Daten der Verbrennungskraftmaschine und des Abgassystems der Verbrennungskraftmaschine ermittelt. In Schritt S12 werden diese Daten bewertet. Bei dem gezeigten Ausführungsbeispiel werden die Daten der Verbrennungskraftmaschine und des Abgassystems der Verbrennungskraftmaschine bewertet.

In Schritt S14 wird anhand der bewerteten Daten geprüft, ob eine vorbestimmte Bedingung für das Messgas vorliegt bzw. erfüllt ist. Bei dem gezeigten Ausführungsbeispiel ist die vorbestimmte Bedingung ein Unterschreiten eines Schwellwerts eines Anteils an Stickoxid in dem Messgas. Mit anderen Worten wird geprüft, ob die Stickoxidkonzentration in dem Messgas bzw. Abgas ausreichend gering ist. Der Schwellwert beträgt 7 % eines Anteils an Ammoniak in dem Messgas.
Wird bei Schritt S14 der Schwellwert überschritten und ist die Stickoxidkonzentration in dem Messgas bzw. Abgas somit nicht ausreichend gering, wird zu Schritt S12 zurückgekehrt. Wird bei Schritt S14 der Schwellwert unterschritten und die Stickoxidkonzentration in dem Messgas bzw. Abgas somit ausreichend gering, wird zu Schritt S16 weitergegangen. Für vorab bestimmte Betriebszustände des Motors und damit des Abgassystems bzw. aus Plausibilisierungen verschiedenen Informationen im Motorsteuergerät kann also abgeleitet werden, ob im Abgasstrang NO und NO₂ vorliegen. Kann dies in Schritt S14 ausgeschlossen werden und wird gleichzeitig ein Signal an der NOx messenden ersten Messeinheit 152 sowie gleichzeitig an der NHs-messenden zweiten Messeinheit detektiert, ist davon auszugehen, dass es sich um NH₃ handelt.

In Schritt S16 werden der erste Messsignalwert der ersten Messeinheit 152 und der zweite Messsignalwert der zweiten Messeinheit 156 miteinander verglichen. Dabei wird ein Abweichungswert des zweiten Messsignalwerts von dem ersten Messsignalwert bestimmt. So kann das Signal der NH₃-messenden zweiten Messeinheit 156 mit dem Signal der NOx-messenden ersten Messeinheit 152 verglichen werden. Weicht das NH₃- Kennlinien-Äquivalent der zweiten Messeinheit 156 vom NH₃-Kennlinien-Äquivalent der ersten Messeinheit 152 ab, wird der Abweichungswert ermittelt. Es wird also der Unterschied des zweiten Messsignalwerts und ersten Messsignalwerts ermittelt bei Vorhandensein von keinen oder nur vernachlässigbaren Mengen an Stickoxiden.

In Schritt S18 wird geprüft, ob der Abweichungswert des zweiten Messsignalwerts von dem ersten Messsignalwert einen vorbestimmten Schwellwert für den Abweichungswert, wie beispielsweise einen Anteil zwischen 10% und 50% und bevorzugt einen Anteil zwischen 10% und 25%, überschreitet. Ist dies nicht der Fall, kann daraus geschlossen werden, dass die zweite Messeinheit 156 nicht oder nur geringfügig gealtert ist und somit kein Korrekturbedarf am Messsignal der zweiten Messeinheit 156 besteht. Bei Unterschreiten des Schwellwerts für den Abweichungswert wird daher zu Schritt S12 zurückgekehrt. Bei Überschreiten des Schwellwerts für den Abweichungswert kann auf eine Alterung der zweiten Messeinheit 156 und somit auf einen Korrekturbedarf am Messsignal der zweiten Messeinheit 156 geschlossen werden. Bei Überschreiten des Schwellwerts für den Abweichungswert wird daher zu Schritt S20 fortgesetzt. In Schritt S20 wird ein Korrekturwert basierend auf dem Abweichungswert gebildet. Der Korrekturwert wird durch Division des zweiten Messsignalwerts mit dem ersten Messsignalwert und anschließendem Bilden des Kehrwerts gebildet.

In Schritt S22 wird der so gebildete Korrekturwert gespeichert, beispielsweise in dem elektronischen Steuergerät 122. In Schritt S24 wird ein korrigiertes zweites Messsignal der zweiten Messeinheit 156 basierend auf dem Korrekturwert und einem zweiten Messsignal der zweiten Messeinheit 156 gebildet. Das zweite korrigierte Messsignal wird durch Multiplizieren des zweiten Messsignals mit dem Korrekturwert gebildet. Mit anderen Worten wird ab sofort der Korrekturwert als Korrekturfaktor auf das NH3-Signal der zweiten Messeinheit 156 angewendet.

Das Verfahren kann umgesetzt werden, indem das Sensorsystem 100 mit einem Motorsteuergerät der Verbrennungskraftmaschine verbunden wird, wobei das Verfahren durchgeführt wird, wenn das Motorsteuergerät ein Triggersignal an das Sensorsystem 100 sendet. Das korrigierte zweite Messsignal kann zur Steuerung oder Regelung einer in das Abgas eingespritzten Menge an Harnstofflösung verwendet werden.

## Patentansprüche

1. Verfahren zur Verringerung von Messfehlern bei der Erfassung von Ammoniak beim Betreiben eines Sensorsystems (100) zum Nachweis mindestens eines Anteils einer Messgaskomponente mit gebundenem Sauerstoff in einem Messgas, insbesondere in einem Abgas einer Verbrennungskraftmaschine, wobei das Sensorsystem (100) mindestens eine erste Messeinheit (152) zum Erfassen von Stickoxid in dem Messgas und eine zweite Messeinheit (156) zum Erfassen von Ammoniak in dem Messgas aufweist, wobei das Verfahren umfasst:
- Erfassen eines ersten Messsignalwerts der ersten Messeinheit (152) bei einer vorbestimmten Bedingung für das Messgas,
- Erfassen eines zweiten Messsignalwerts der zweiten Messeinheit (156) bei der vorbestimmten Bedingung für das Messgas,
- Bestimmen eines Abweichungswerts des zweiten Messsignalwerts von dem ersten Messsignalwert,
- Bilden eines Korrekturwerts basierend auf dem Abweichungswert und
- Bilden eines korrigierten zweiten Messsignals der zweiten Messeinheit basierend auf dem Korrekturwert und einem zweiten Messsignal der zweiten Messeinheit,
wobei die vorbestimmte Bedingung darin besteht, dass ein Anteil an Stickoxid in dem Messgas 7% eines Anteils an Ammoniak in dem Messgas unterschreitet.

2. Verfahren nach Anspruch 1 , wobei der Korrekturwert durch Division des zweiten Messsignalwerts mit dem ersten Messsignalwert und anschließendem Bilden des Kehrwerts gebildet wird.

3. Verfahren nach einem der Ansprüche 1 oder 2, wobei das zweite korrigierte Messsignal durch Multiplizieren des zweiten Messsignals mit dem Korrekturwert gebildet wird.

4. Verfahren nach einem der Ansprüche 1 oder 3, wobei der Korrekturwert nur gebildet wird, falls der Abweichungswert des zweiten Messsignalwerts von dem ersten Messsignalwert einen vorbestimmten Schwellwert für den Abweichungswert, einen Anteil zwischen 10% und 50% und bevorzugt einen Anteil zwischen 10% bis 25%, überschreitet.

5. Verfahren nach einem der Ansprüche 1 bis 4, wobei die erste Messeinheit (152) und die zweite Messeinheit (156) in einem einzigen Sensorelement angeordnet sind.

6. Verfahren nach einem der Ansprüche 1 bis 5, wobei die erste Messeinheit (152) in einem ersten Sensorelement (110) angeordnet ist und die zweite Messeinheit (156) in einem zweiten Sensorelement (154) angeordnet ist, wobei sich das erste Sensorelement (110) von dem zweiten Sensorelement (154) unterscheidet, wobei das erste Sensorelement (110) und das zweite Sensorelement (154) in einem einzigen Sensor angeordnet sind.

7. Verfahren nach einem der Ansprüche 1 bis 6, wobei die erste Messeinheit (152) in einem ersten Sensorelement (110) angeordnet ist und die zweite Messeinheit (156) in einem zweiten Sensorelement (154) angeordnet ist, wobei sich das erste Sensorelement (110) von dem zweiten Sensorelement (154) unterscheidet, wobei das erste Sensorelement (110) in einem ersten Sensor angeordnet ist und das zweite Sensorelement (154) in einem zweiten Sensor angeordnet ist, wobei sich der erste Sensor von dem zweiten Sensor unterscheidet, wobei der erste Sensor und der zweite Sensor benachbart zueinander in dem Messgas angeordnet werden.

8. Verfahren nach einem der Ansprüche 1 bis 7, wobei das Messgas Abgas einer Verbrennungskraftmaschine ist, wobei das Sensorsystem (100) mit einem Motorsteuergerät der Verbrennungskraftmaschine verbunden wird, wobei das Verfahren durchgeführt wird, wenn das Motorsteuergerät ein Triggersignal an das Sensorsystem (100) sendet.

9. Verfahren nach einem der Ansprüche 1 bis 8, wobei das Messgas Abgas einer Verbrennungskraftmaschine ist, wobei das korrigierte zweite Messsignal zur Steuerung oder Regelung einer in das Abgas eingespritzten Menge an Harnstofflösung verwendet wird.

10. Sensorsystem (100) zum Nachweis mindestens eines Anteils einer Messgaskomponente mit gebundenem Sauerstoff in einem Messgas, insbesondere in einem Abgas einer Verbrennungskraftmaschine, umfassend mindestens eine erste Messeinheit (152) zum Erfassen von Stickoxid in dem Messgas und eine zweite Messeinheit (156) zum Erfassen von Ammoniak in dem Messgas, wobei das Sensorsystem (100) weiterhin ein elektronisches Steuergerät (122) aufweist, das ein elektronisches Speichermedium umfasst, auf welchem ein Computerprogramm gespeichert ist, welches eingerichtet ist, jeden Schritt des Verfahrens nach einem der vorhergehenden Ansprüche durchzuführen.

## Claims

1. Method for reducing measurement errors when detecting ammonia during the operation of a sensor system (100) for identifying at least a proportion of a measurement gas component with bound oxygen in a measurement gas, in particular in an exhaust gas of a combustion engine, wherein the sensor system (100) has at least a first measuring unit (152) for detecting nitrogen oxide in the measurement gas and a second measuring unit (156) for detecting ammonia in the measurement gas, the method comprising the steps of:
- recording a first measurement signal value of the first measuring unit (152) in the case of a predetermined condition for the measurement gas,
- recording a second measurement signal value of the second measuring unit (156) in the case of the predetermined condition for the measurement gas,
- determining a deviation value of the second measurement signal value from the first measurement signal value,
- forming a correction value on the basis of the deviation value and
- forming a corrected second measurement signal of the second measuring unit on the basis of the correction value and a second measurement signal of the second measuring unit,
wherein the predetermined condition consists of a proportion of nitrogen oxide in the measuring gas being less than 7% of a proportion of ammonia in the measurement gas.

2. Method according to Claim 1, wherein the correction value is formed by dividing the second measurement signal value by the first measurement signal value and subsequently forming the reciprocal.

3. Method according to either of Claims 1 and 2, wherein the second corrected measurement signal is formed by multiplying the second measurement signal by the correction value.

4. Method according to either of Claims 1 and 3, wherein the correction value is only formed if the deviation value of the second measurement value signal from the first measurement signal value exceeds a predetermined threshold value for the deviation value, a proportion between 10% and 50% and preferably a proportion between 10% and 25%.

5. Method according to any one of Claims 1 to 4, wherein the first measuring unit (152) and the second measuring unit (156) are arranged in a single sensor element.

6. Method according to any one of Claims 1 to 5, wherein the first measuring unit (152) is arranged in a first sensor element (110) and the second measuring unit (156) is arranged in a second sensor element (154), wherein the first sensor element (110) differs from the second sensor element (154), wherein the first sensor element (110) and the second sensor element (154) are arranged in a single sensor.

7. Method according to any one of Claims 1 to 6, wherein the first measuring unit (152) is arranged in a first sensor element (110) and the second measuring unit (156) is arranged in a second sensor element (154), wherein the first sensor element (110) differs from the second sensor element (154), wherein the first sensor element (110) is arranged in a first sensor and the second sensor element (154) is arranged in a second sensor, wherein the first sensor differs from the second sensor, wherein the first sensor and the second sensor are arranged adjacently to one another in the measurement gas.

8. Method according to any one of Claims 1 to 7, wherein the measurement gas is exhaust gas from a combustion engine, wherein the sensor system (100) is connected to an engine control unit of the combustion engine, wherein the method is carried out when the engine control unit transmits a trigger signal to the sensor system (100).

9. Method according to any one of Claims 1 to 8, wherein the measurement gas is exhaust gas from a combustion engine, wherein the corrected second measurement signal is used for open-loop or closed-loop control of an amount of urea solution injected into the exhaust gas.

10. Sensor system (100) for identifying at least a proportion of a measurement gas component with bound oxygen in a measurement gas, in particular in an exhaust gas of a combustion engine, comprising at least a first measuring unit (152) for detecting nitrogen oxide in the measurement gas and a second measuring unit (156) for detecting ammonia in the measurement gas, wherein the sensor system (100) furthermore has an electronic controller (122) which comprises an electronic storage medium, stored on which there is a computer program configured to carry out every step of the method according to any one of the preceding claims.

## Revendications

1. Procédé pour réduire des erreurs de mesure lors de la détection d'ammoniac lors du fonctionnement d'un système capteur (100) destiné à déceler
au moins une proportion d'une composante gazeuse à mesurer avec de l'oxygène lié dans un gaz à mesurer, notamment dans un gaz d'échappement d'un moteur à combustion interne, le système capteur (100) possédant au moins une première unité de mesure (152) destinée à détecter de l'oxyde d'azote dans le gaz à mesurer et une deuxième unité de mesure (156) destinée à détecter de l'ammoniac dans le gaz à mesurer, le procédé comprenant :
- acquisition d'une première valeur de signal de mesure de la première unité de mesure (152) en présence d'une condition prédéterminée pour le gaz à mesurer,
- acquisition d'une deuxième valeur de signal de mesure de la deuxième unité de mesure (156) en présence de la condition prédéterminée pour le gaz à mesurer,
- détermination d'une valeur d'écart entre la deuxième valeur de signal de mesure et la première valeur de signal de mesure,
- formation d'une valeur de correction sur la base de la valeur d'écart et
- formation d'un deuxième signal de mesure corrigé de la deuxième unité de mesure sur la base de la valeur de correction et d'un deuxième signal de mesure de la deuxième unité de mesure,
la condition prédéterminée consistant en ce qu'une proportion d'oxyde d'azote dans le gaz à mesurer devient inférieure à 7 % d'une proportion d'ammoniac dans le gaz à mesurer.

2. Procédé selon la revendication 1, la valeur de correction étant formée par la division de la deuxième valeur de signal de mesure par la première valeur de signal de mesure et ensuite formation de la valeur réciproque.

3. Procédé selon l'une des revendications 1 ou 2, le deuxième signal de mesure corrigé étant formé en multipliant le deuxième signal de mesure par la valeur de correction.

4. Procédé selon l'une des revendications 1 ou 3, la valeur de correction n'étant formée que dans le cas où la valeur d'écart entre la deuxième valeur de signal de mesure et la première valeur de signal de mesure dépasse une valeur de seuil prédéterminée pour la valeur d'écart, une proportion entre 10 % et 50 % et de préférence une proportion entre 10 % et 25 %.

5. Procédé selon l'une des revendications 1 à 4, la première unité de mesure (152) et la deuxième unité de mesure (156) étant disposées dans un élément capteur unique.

6. Procédé selon l'une des revendications 1 à 5, la première unité de mesure (152) étant disposée dans un premier élément capteur (110) et la deuxième unité de mesure (156) étant disposée dans un deuxième élément capteur (154), le premier élément capteur (110) se différenciant du deuxième élément capteur (154), le premier élément capteur (110) et le deuxième élément capteur (154) étant disposées dans un capteur unique.

7. Procédé selon l'une des revendications 1 à 6, la première unité de mesure (152) étant disposée dans un premier élément capteur (110) et la deuxième unité de mesure (156) étant disposée dans un deuxième élément capteur (154), le premier élément capteur (110) se différenciant du deuxième élément capteur (154), le premier élément capteur (110) étant disposé dans un premier capteur et le deuxième élément capteur (154) étant disposé dans un deuxième capteur, le premier capteur se différenciant du deuxième capteur, le premier capteur et le deuxième capteur étant disposés adjacents l'un de l'autre dans le gaz à mesurer.

8. Procédé selon l'une des revendications 1 à 7, le gaz à mesurer étant un gaz d'échappement d'un moteur à combustion interne, le système capteur (100) étant relié à un contrôleur de moteur du moteur à combustion interne, le procédé étant mis en œuvre lorsque le contrôleur de moteur envoie un signal de déclenchement au système capteur (100).

9. Procédé selon l'une des revendications 1 à 8, le gaz à mesurer étant un gaz d'échappement d'un moteur à combustion interne, le deuxième signal de mesure corrigé étant utilisé pour commander ou pour réguler une quantité de solution d'urée injectée dans le gaz d'échappement.

10. Système capteur (100) destiné à déceler au moins une proportion d'une composante gazeuse à mesurer avec de l'oxygène lié dans un gaz à mesurer, notamment dans un gaz d'échappement d'un moteur à combustion interne, comprenant au moins une première unité de mesure (152) destinée à détecter de l'oxyde d'azote dans le gaz à mesurer et une deuxième unité de mesure (156) destinée à détecter de l'ammoniac dans le gaz à mesurer, le système capteur (100) possédant en outre un contrôleur électronique (122), lequel comprend un support d'enregistrement électronique sur lequel est enregistré un programme informatique, lequel est conçu pour exécuter chaque étape du procédé selon l'une des revendications précédentes.
